(19) Europäisches Patentamt
European Patent Office
Office européen des brevets

(11) Numéro de publication: **0 011 057**
**B1**

(12) **FASCICULE DE BREVET EUROPEEN**

(45) Date de publication du fascicule du brevet: **09.02.83**

(21) Numéro de dépôt: **79870020.9**

(22) Date de dépôt: **22.08.79**

(51) Int. Cl.³: **C 07 D 471/12,**
C 07 D 471/04,
C 07 D 487/04

(54) **Procédé de synthèse de la vincadifformine et des dérivés apparentés à celle-ci, produits intermédiaires obtenus pendant cette synthèse et procédé pour leur préparation.**

(30) Priorité: **24.08.78 US 936454**

(43) Date de publication de la demande:
**14.05.80 Bulletin 80/10**

(45) Mention de la délivrance du brevet:
**09.02.83 Bulletin 83/6**

(84) Etats contractants désignés:
**AT BE CH DE FR GB IT LU NL SE**

(56) Documents cités:
**AT - B - 260 257**
**FR - A - 1 524 495**
**FR - A - 1 524 830**
**US - A - 3 553 232**
**US - A - 4 154 943**

(73) Titulaire: **OMNICHEM Société anonyme**
**12 Avenue de Broqueville**
**B-1150 Bruxelles (BE)**

(72) Inventeur: **Kuehne, Martin, Eric**
**169, South Cove Road**
**Burlington, Vermont 05401 (US)**

(74) Mandataire: **de Kemmeter, François et al,**
**Cabinet Bede 13, avenue Antoine Depage**
**B-1050 Bruxelles (BE)**

**EP 0 011 057 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé de synthèse de la vincadifformine et des dérivés apparentés à celle-ci, produits intermédiaires obtenus pendant cette synthèse et procédé pour leur préparation

La présente invention est relative à un procédé de synthèse de la vincadifformine et de ses dérivés et concerne également les intermédiaires de cette synthèse.

Les composés préparés par le procédé selon l'invention répondent à la formule générale I

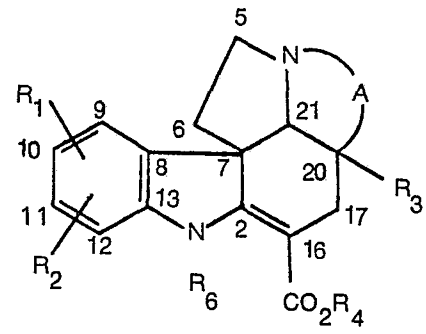

(I)

| | |
|---|---|
| Ia-h | $R_6$=H |
| Ia | $R_1=R_2=H$, $R_3=CH_2CH_3$, $R_4=CH_3$ A=$—(CH_2)_3—$ |
| Ib | $R_1$=11—$OCH_3$, $R_2$=H, $R_3=CH_2—CH_3$, $R_4=CH_3$ A=$—(CH_2)_3—$ |
| Ic | $R_1=R_2=H$, $R_3=CH_2—CH_3$, $R_4=CH_3$ A=$—CH_2—CH(OH)—CH_2—$ |
| Id | $R_1=R_2=H$, $R_3=CH_2—CH_3$, $R_4=CH_3$ >N—A—=—N—CH($CH_2$OH)—$CH_2$ |
| Ie | $R_1=R_2=H$, $R_3$=H, $R_4=CH_3$ A=$—CH_2—CH(CH_2CH_3)—CH_2—$ |
| If | $R_1$=10—Cl, $R_2$=H, $R_3=CH_2—CH_3$, $R_4=CH_3$, A=$—(CH_2)_3—$ |
| Ig | $R_1$=10—Br, $R_2$=H, $R_3=CH_2—CH_3$, $R_4=CH_3$, A=$—(CH_2)_3—$ |
| Ih | $R_1$=10—$OCH_3$, $R_2$=H, $R_3=CH_2—CH_3$, $R_4=CH_3$ A=$—(CH_2)_3—$ |

Dans la formule générale donnée ci-dessus, $R_1$ et $R_2$ sont égaux ou différents étant choisis parmi l'hydrogène, les groupes hydroxy, acyloxy, carbamate, halogéno, alkoxy inférieur, alkyle inférieur, $R_3$, $R_4$ et $R_6$ représentent alkyle inférieur ou hydrogène ou une combinaison de ces radicaux, A représente une chaîne alkyle contenant 2 ou 3 atomes de carbone et qui peut être substituée par un ou plusieurs groupes hydroxy, alkyle ou hydroxyalkyle inférieur.

La numérotation des positions de la vincadifformine et de ses dérivés est telle que préconisée par Le Men et Taylor dans Experientia 1965, *21,* 500.

Les composés de formules Ia à Ih dont la définition a été donnée ci-dessus forment quelques exemples représentatifs de composés que l'on prépare suivant la présente invention.

La vincadifformine de formule Ia est un alcaloïde, produit de départ de la préparation des alcaloïdes du groupe de la vincamine comme décrit dans les brevets belges No 772.005 et 848.475.

La vincamine et certains de ses dérivés sont des alcaloïdes bien connus qui servet en médecine humaine en tant que médicaments psychotropes de grande efficacité relativement peu toxiques.

De plus, il a été montré que le réarrangement de la vincadifformine menant à la vincamine peut être appliqué à un grand nombre de dérivés semblables et peut donner lieu à des composés apparentés à la vincamine (voir français No. 76 22275 et brevet belge No. 816.692).

Deux procédés de synthèse totale de la vincadifformine ont déjà été décrits dans la littérature par S. Kutney et al, J. Amer. Chem. Soc. 90, 3891, 1968 et J. V. Laronze et al, Tetrahedron Letters 491, 1974.

Un autre procédé de synthèse totale de la vincadifformine est décrit et mis sous protection dans le brevet américain No 4.154.943, publié postérieurement à la date de priorité revendiquée pour la présente demande de brevet.

La 11-méthoxy vincadifformine (ervincéine) de formule Ib est un alcaloïde que l'on trouve dans Vinca Erecta et qui a été décrit par D. A. Rakhimov, V. M. Malikov, M. R. Yagudaev et S. N. Yunusov (Khim, prir. Soedin. 226, 1970).

La pseudo-vinadifformine de formule Ie est un alcaloïde que l'on trouve dans différentes Apocynacées et qui a été obtenue également par hémisynthèse (J. P. Kutney, E. Piers et R. T. Brown, J. Amer. Chem. Soc. 92, 1700, 1970).

Le procédé de la présente invention utilise, à titre d'intermédiaire, de nouvelles hexahydro azépino 4,5-b indoles dicarboalkoxylés en position 5.

Certains composés possédant le squelette polycyclique des azépino 4,5-b indoles ont déjà été décrits, notamment par Ulrich Renner (brevet américain 3.525.750) et Hester et al. (J. Med. Chem. *11*, 101, 1968). Aucun composé comportant un groupe carboalkoxy en $C_5$ n'a cependant été relevé et aucun auteur n'a envisagé l'utilisation d'hexahydroazépino indoles pour la synthèse de dérivés pentacycliques du type vincadifformine.

La présente invention vise à procurer un procédé d'obtention de la vincamine et de composés polycycliques apparentés avec un rendement élevé, en réduisant le nombre d'étapes intermédiaires et en partant de réactifs bon marché.

A cet effet, on utilise comme matière première de départ une N-benzyltétrahydro-γ-carboline ou 2-benzyl-1,2,3,4-tetrahydro-γ-carboline de formule générale III

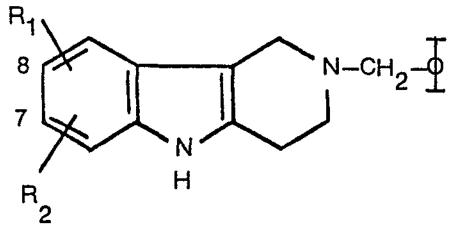

(III)

| | |
|---|---|
| IIIa | $R_1$=$R_2$=H |
| IIIb | $R_1$=7—$OCH_3$; $R_2$=H |
| IIIc | $R_1$=8—$OCH_3$; $R_2$=H |
| IIId | $R_1$=8—Cl; $R_2$=H |
| IIIe | $R_1$=8—Br; $R_2$=H |

dans laquelle $R_1$ et $R_2$ ont la signification donnée plus haut et Φ représente phényle, que l'on obtient avantageusement à partir d'une N-benzylpipéridone, produit bon marché, de formule

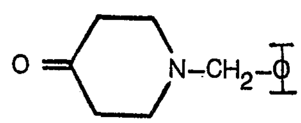

(IIa)

en passant par une N-benzylpipéridone phénylhydrazone de formule générale IIb

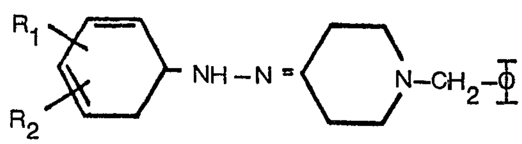

(IIb)

et en faisant réagir ce dernier composé dans les conditions de la "Synthèse Indolique de Fischer".

Conformément à l'invention, la N-benzyltétrahydro-γ-carboline (III) est tranformée, dans un premier stade, par action de l'hypochlorite de t-butyle ou d'un agent de chloration similaire en chloro-indolénine de formule

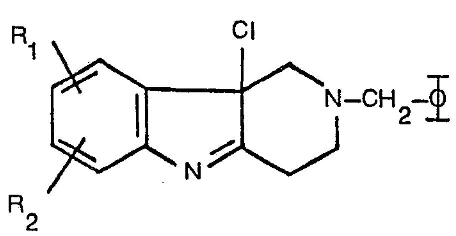

(IV)

qui est immédiatement traitée avec du thallium (sodium ou autre métal) malonate de di-alkyle de préférence diéthyle ou diméthyle, pour donner un 3-benzyl-1,2,3,4,5,6-hexahydroazépino (4,5-b) indole-5,5-di(carboxylate d'alkyle) de formule

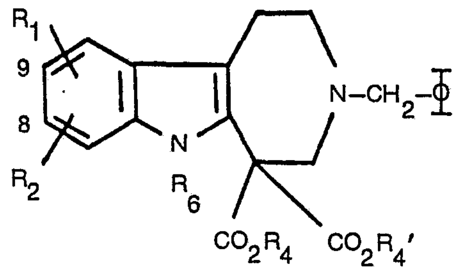

(V)

Va-e $R_4=R_4'=CH_3$; $R_6=H$

Va $R_1=R_2=H$

Vb $R_1=8—OCH_3$; $R_2=H$

Vc $R_1=H$; $R_2=9—OCH_3$

Vd $R_1=H$; $R_2=9—Cl$

Ve $R_1=H$; $R_2=9—Br$

dans laquelle $R_1$, $R_2$ et $R_4$ sont tels que définis précédemment. $R_4'$ est un groupe alkyle inférieur par exemple méthyle, éthyle ou t-butyle.

Pour cette condensation, on peut utiliser n'importe quel solvant inerte dans les conditions de réaction. Le benzène et le toluène conviennent particulièrement bien à cet effet.

Si on le désire le composé (V) ($R_6=H$) peut être N-alkylé en utilisant les modes opératoires connus de N-alkylation des dérivés de l'indole, pour donner (V) où $R_6$ est un alkyle inférieur.

Dans un deuxième stade, le dérivé (V) est hydrogéné en présence d'un catalyseur, par exemple le Pd/C à 5%, pour donner un 1,2,3,4,5,6-hexahydroazépino(4,5-b)indole-5,5-dicarboxylate d'alkyle) de formule

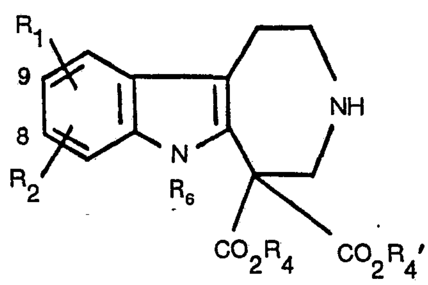

(VI)

VIa-e $R_4=R_4'=CH_3$; $R_6=H$

VIa $R_1=R_2=H$

VIb $R_1=8—OCH_3$; $R_2=H$

VIc $R_1=H$; $R_2=9—OCH_3$

VId $R_1=H$; $R_2=9—Cl$

VIe $R_1=H$; $R_2=9—Br$

Dans un troisième stade, le dérivé VI est condensé avec un aldéhyde VII "fonctionnalisé" pour donner lieu à la vincadifformine ou un dérivé de formule I apparenté à celle-ci.

Par aldéhyde fonctionnalisé, on entend un aldéhyde dont un dérivé énamine tertiaire peut subir une N-alkylation intromoléculaire.

Ces composés sont choisis parmi les aldéhydes halogénés, arylsulfonates, alkylsulfonates ou époxydes qui comportent une chaîne de 4 ou 5 atomes de carbone. Ces aldéhydes sont éventuellement substitués par un ou plusieurs groupes hydroxy, alkyle ou hydroxyalkyle dont la chaîne alkyle contient de 1 à 7 atomes de carbone. Ces aldéhydes comportent donc de 4 à 12 atomes de carbone au total.

Des exemples de ces aldéhydes sont le 5-bromo-2-éthyl-pentanal (VIIa), le 5-chloro-2-éthyl-pentanal, le 5-sulfo-méthoxy-2-éthyl-pentanal, le 4-bromométhyl-hexanal (VIIb), le 2-éthyl-4-oxyranyl-pentanal (VIIc).

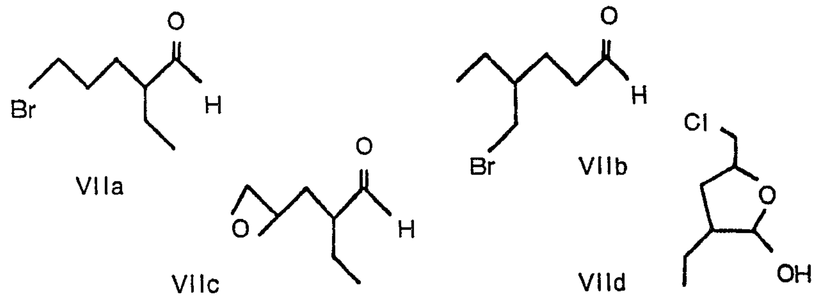

VIIa VIIb VIIc VIId

En présence d'un groupe hydroxyle supplémentaire ces aldéhydes peuvent prendre une forme hémi-acétalisée comme VIId.

En cas de réaction d'un halogénoaldéhyde avec un azépino-indole VI, on peut isoler le composé intermédiaire de formule générale VIII. Dans cette formule, $X^-$ représente un ion halogénure, par exemple bromure. Cet ion peut être échangé pour donner lieu au dérivé pour lequel $X^-$ est un ion tétraphénylborate.

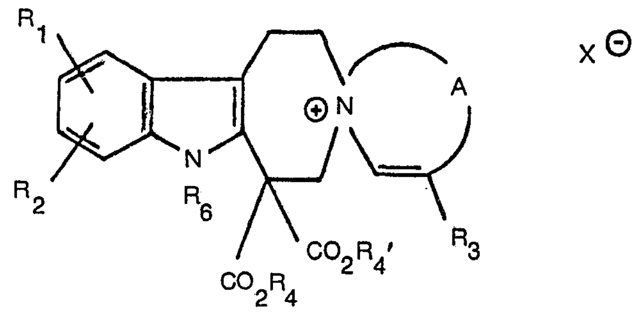

VIII

Pour effectuer la condensation (VI + aldéhyde en passant par VIII) on a trouvé préférable d'utiliser un solvant tel que le méthanol sec mais on peut également utiliser d'autres solvants inertes dans les conditions de la réaction.

La température de la réaction peut varier de −20°C au point d'ébullition du milieu réactionnel. Les gammes de températures préférées vont de 20°C à 40°C.

En variante, on peut effectuer la monodécarboxylation des intermédiaires V ou VI pour obtenir une amine monoestérifiée (IX) que l'on peut transformer en vincadifformine ou un dérivé apparenté de celle-ci selon la procédé décrit et mis sous protection dans le brevet américain No 4.154.943.

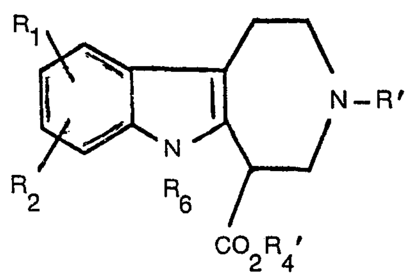

IX

| | |
|---|---|
| IXa-e | $R_4$=$CH_3$; $R_6$=H |
| IXa | $R_1$=$R_2$=H |
| IXb | $R_1$=8—$OCH_3$; $R_2$=H |
| IXc | $R_1$=H; $R_2$=9—$OCH_3$ |
| IXd | $R_1$=H; $R_2$=9—Cl |
| IXe | $R_1$=H; $R_2$=9—Br |

Le 5-bromo-2-éthylpentanal ou 1-bromo-4-formylhexane utilisé dans la dernière étape du procédé peut se préparer comme suit:

On effectue l'acétylation du 4-formylhexanoate de méthyle ou d'éthyle pour former le 4-diméthoxyméthylhexanoate de méthyle ou d'éthyle respectivement, que l'on réduit à l'aide d'hydrure de lithium-aluminium en 4-diméthoxy-méthyl-1-hexanol ou 4-diéthoxy-méthyl-1-hexanol respectivement. L'alcool ainsi obtenu subit une déshydroxybromation pour donner lieu au 1-bromo-4-diméthoxyméthyl-hexane et au 1-bromo-4-diéthoxyméthyl-hexane respectivement que l'on hydrolyse pour former le 1-bromo-4-formyl-hexane ou 5-bromo-2-éthylpentanal voulu.

Un autre mode de préparation du 5-bromo-2-éthyl-pentanal est également décrit dans la publication de W. Oppolzer, H. Hauth, P. Pfaffli. R. Wenger, Helv. *60,* 1861 (1977).

Les exemples suivants illustrent de façon non limitatative les caractéristiques de l'invention.

Exemple 1

N-benzyl-4-pipéridone phényl-hydrazone (formule IIb)

A une solution de 600 ml d'un mélange 1 : 1 d'eau et d'éthanol, on ajoute 38 g (0,2 mole) de N-benzyl-4-pipéridone (formule IIa) et 32 g (0,22 mole) de chlorhydrate de phénylhydrazine. On agite la solution 48 heures à température ambiante et on l'alcalinise en y ajoutant du carbonate de potassium en solution saturée, l'addition devant se faire à une vitesse qui permet de contrôler la formation de mousse. le produit se sépare sous forme d'une huile que l'on recueille. La couche aqueuse subit une extraction à l'acétate d'éthyle et les fractions organiques, après avoir été rassemblées, sont séchées sur du $MgSO_4$ et le solvant est chassé sous vide. On ajoute 100 ml d'hexane et on le chasse ensuite sous vide pour aider à éliminer les restes de solvants polaires. On ajoute au produit qui cristallise 200 ml d'hexane et après 20 minutes, une filtration sous vide donne 50 g (87%) de dérivé de phénylhydrazone (de formule IIb) convenant à l'utilisation dans l'étape suivante. Le produit peut être recristallisé dans l'hexane (P. F. 80—81) [litt. 78—79°C selon N. P. Buu-Hoi, Odette Roussel et P. Jacquignon, J. Chem. Soc. 708 (1964)].

Exemple 2

N-benzyl-tétrahydro-$\gamma$-carboline (formule IIIe)

On ajoute 49 g (0,18 mole) de N-benzylpipéridone phénylhydrazone (IIb) en agitant à 250 ml d'acide acétique glacial contenant 7% en poids d'acide chlorhydrique anhydre. La solution tourne immédiatement au rouge foncé et devient très chaude. On continue d'agiter jusqu'à ce que la solution se soit refroidie jusqu'à la température ambiante (environ 15 à 20 minutes). On alcalinise ensuite la solution en y ajoutant prudemment une solution aqueuse saturée de carbonate de potassium et on l'extrait à l'aide de dichlorométhane. Après séchage sur du $MgSO_4$ et après avoir enlevé le solvant, on recristallise le résidu. On lave ce produit à l'éthanol et on le recristallise dans l'éthanol pour obtenir 29,5 g (65%) de N-benzyl-tétrahydro-$\gamma$-carboline.

P.F. 158—159°C (lit. 161°C, même référence qu'à l'exemple 1).

RMN ($CDCl_3$) $\delta$

2,6—3,0 (4H, m), 3,75 (2H, s), 3,81 (2H, s), 7,0—7,5 (9H, m) 7,8—8,0 (1H, large singlet).

Exemple 3

2-benzyl-7-methoxy,1,2,3,4-tétrahydro-$\gamma$-carboline (IIIb)

On agite une solution de 5,0 g (0,0286 mole) de chlorhydrate de m-méthoxyphénylhydrazine et 5,04 g (0,0267 mole) de N-benzyl-4-pipéridone dans 175 ml de méthanol aqueux à 50%, sous $N_2$ pendant 72 heures à 20°C. On alcalinise la solution en y ajoutant du $K_2CO_3$ en solution aqueuse saturée en sorte que l'hydrazone se sépare sous forme d'une huile brune. On transvase le mélange, avec de l'acétate d'éthyle, dans un entonnoir à robinet de 1 l, on sépare la couche aqueuse et on l'extrait deux fois au moyen d'acétate d'éthyle. Après avoir rassemblé les couches organiques on les extraite deux fois au moyen d'une saumure saturée, on sèche sur $MgSO_4$, on filtre et on enlève le solvant pour obtenur une huile visqueuse et brune. L'hydrazone brute est dissoute dans 50 ml d'acide acétique et on y ajoute 140 ml d'acide acétique saturé de HCl tout en agitant vigoureusement. On agite la solution sous azote dans un bain d'huile à 90°C pendant 30 minutes, on la refroidit à 20°C et on l'alcalinise en ajoutant du $K_2CO_3$ en solution aqueuse saturée tout en agitant vigoureusement (l'addition de 100 ml d'acétate d'éthyle aide à contrôler la formation de mousse). Le mélange est transvasé dans un entonnoir à robinet de 1 l avec 200 ml d'acétate d'éthyle et 100 ml d'eau et la couche aqueuse est séparée et extraite deux foix à l'acétate d'éthyle. On rassemble les couches organiques, on les lave deux fois à la saumure saturée, on sèche sur $MgSO_4$ et on filtre. L'enlèvement du solvant laisse une gomme huileuse brune qui cristallise par trituration avec de l'éthanol à 95%. En recristallisant dans le

même solvant et dans le méthanol on obtient 2,75 g (35%) de cristaux blanchâtres de composé (IIIb).

P.F. 172—173°C
RMN [$CDCl_3$+DMSO($D_6$)] $\delta$
10,30 (bs, 1H), 7,30 (m, 5H), 7,07 (d, 1H, J=8Hz), 6,75 (d, 1H, J=2Hz), 6,53 (d de d, 1H, J=8Hz, 2Hz), 3,71 (s, 3H), 3,70 (s, 2H), 3,53 (s, 2H), 2,75 (s, 4H);
IR (KBr):
3410, 1640, 1610, 1580 $cm^{-1}$
Spectre de masse (80eV) m/e 292 ($M^+$), 173 (base)
Analyse calc. pour $C_{19}H_{20}N_2$

| | | | |
|---|---|---|---|
| | C 78,09% | H 6,90% | N 9,58% |
| trouvé: | C 78,05% | H 7,04% | N 9,58% |

## Exemple 4

3-benzyl-1,2,3,4,5,6-hexahydroazépino(4,5-b)indole-5,5-di(carboxylate de méthyle) (formule Va)

A une solution de 0,25 g (0,95 millimole) de N-benzyl-tétrahydro-$\gamma$-carboline (formule IIIa) et 110 $\mu$l de triéthylamine dans 7 ml de benzène sec, on ajoute goutte à goutte, tout en agitant à 5°C sous azote, 110 $\mu$l (0,954 millimole) de tertio-butylhypochlorite. On agite le mélange à 5°C pendant 1 h.30 minutes puis on le verse dans environ 3 ml d'eau froide dans une boule à décanter. Après avoir agité modérément on laisse les couches se séparer et on jette la couche aqueuse. On sèche la couche benzénique par filtration à travers un cône de sulfate de sodium contenu dans un papier séparateur de phases. La boule à décanter et le cône filtrant sont lavés à l'aide de benzène sec et le filtrat benzénique est évaporé sous vide jusqu'à environ 3 ml. On ajoute du benzène sec pour atteindre un volume d'environ 7 ml puis on ajoute également 0,335 g (1 millimole) de thallium malonate de diméthyle et la solution ainsi obtenue est chauffée au reflux sous agitation vigoureuse pendant 23 heures sous azote. Le milieu de réaction est ensuite refroidi à température ambiante et est filtré à travers un papier à fibres de verre, puis le benzène est évaporé sous vide. Le résidu est repris dans du dichlorométhane et est déposé sur une colonne de $SiO_2$ (1,5 cm x 30 cm). L'élution avec du dichlorométhane donne 0,195 g (52%) d'un produit légèrement coloré dont les spectres IR et RMN sont identiques à ceux du produit obtenu avec un rendement de 46% à partir de N-benzyl-tétrahydro-$\gamma$-carboline par la même séquence réactionnelle. En recristallisant dans le méthanol, on obtient des cristaux blancs de produit (Va).

P.F. 166—168°C
RMN ($CDCl_3$ $\delta$):
2,97 (s, 4H), 3,8 (s, 2H), 3,88 (s, 6H), 3,93 (s, 2H), 7,24—7,8 (m, 9H), 8,64 (large s, 1H)
IR (KBr, $\nu_{max}$):
3510, 3010, 3000, 2950, 2940, 2920, 2880, 2825, 1750, 1705, 1490, 1450, 1440, 1435, 1345, 1320, 1285, 1275, 1255, 1240, 1220, (large), 1185, 1165, 1145, 1135, 1125, 1110, 1090, 1075, 1055, 1035, 980, 960, 935, 700, 690, 670, 635 $cm^{-1}$
Spectre de masse (m/e) 392 ($M^+$)
Analyse calc. pour $C_{23}H_{24}N_2O_4$:

| | | | |
|---|---|---|---|
| | C 70, 39% | H 6,16% | N 7,14% |
| Trouvé: | C 70,12% | H 6,25% | N 6,91% |

## Exemple 5

3-benzyl-8-méthoxy-1,2,3,4,5,6-hexahydroazépino(4,5-b)indole-5,5-di(carboxylate de méthyle) (Vb)

A une solution de 2,5 g (8,56 millimole) de méthoxy-tétrahydro-$\gamma$-carboline (IIIb) et 1,07 ml de triéthylamine dans 60 ml de dichlorométhane, en agitant sous $N_2$ à —78°C, on ajoute sous la surface du liquide au moyen d'une seringue, 5 ml d'une solution de 1,2 ml de t-butylhypochlorite dans 4,4 ml de dichlorométhane. On agite le mélange 15 minutes puis on le laisse se réchauffer à 20°C. On enlève le solvant sous vide, ce qui laisse une huile brune visqueuse. Une chromatographie en couche mince ($Al_2O_3$, $CH_2Cl_2$) montre une nette transformation en chloroindolénine.

Le produit brut est repris dans 50 ml de benzène sec, on y ajoute 3,45 g (0,0103 mole, 1,2 X) de thallium malonate de diméthyle et on agite vigoureusement la suspension ainsi formée, sous azote, 2 heures à 20°C puis encore 3,5 h.au reflux. On laisse le mélange se refroidir à 20°C et on y ajoute une solution saturée de NaCl pour précipiter le TlCl. On filtre le mélange sur du papier à fibres de verre (GF/A) et on rince le flacon et le gâteau de filtration à l'aide de benzène. On sépare la couche organique et on extrait la couche aqueuse à l'aide de benzène. Les couches benzéniques sont rassemblées, lavées à l'aide de saumure saturée, séchée sur $MgSO_4$ et filtrées et le solvant est chassé sous vide pour donner lieu à un produit solide brunâtre. Une recristallisation dans le méthanol donne 2,19 g de cristaux blancs. La chromatographie ($SiO_2$, MeOH à 5% dans $CH_2Cl_2$) de la liqueur mère donne des fractions contenant le produit désiré qui par combinaison des fractions, enlèvement du solvant et recristallisation dans le méthanol avec ensemencement, produit un supplément de 0,36 g de cristaux blanchâtres de produit (Vb).

Rendement total:
2,56 g (71%)
RMN ($CDCl_3$,δ):
8,26 (large s, 1H), 7,28 (m, 6H), 6,70 (d de d, 2H), 3,78 (s, 2H), 3,76 (s, 3H), 3,72 (s, 6H), 3,66 (s, 2H), 2,84 (s, 4H)
IR (KBr):
3462, 1790, 1635, 1575, 1515, 1905, 1255, 1220, 1210 $cm^{-1}$
Spectre de masse (80eV) m/e:
422 ($M^+$). Un échantillon pour analyse, recristallisé dans le méthanol fond à 132—134°C.
Analyse calc. pour $C_{24}H_{26}N_2O_5$

| | | | |
|---|---|---|---|
| Calc.: | C 68,23% | H 6,20% | N 6,63% |
| Trouvé: | C 67,9% | H 6,40% | N 6,45% |

Exemple 6

1,2,3,4,5,6-hexahydroazépino(4,5-b)indole-5,5-di(carboxylate de méthyle) (formule VIa)

A 1,6 g (4,08 millimole) du dérivé diester de benzylamine de formule Va en solution dans 50 ml d'acide acétique, on ajoute 0,2 g de catalyseur de palladium à 5% sur de charbon. L'hydrogénation à pression atmosphérique avec agitation vigoureuse montre la fin de l'absorption d'hydrogène après 3 heures. La solution est filtrée à travers un papier comportant des fibres de verre et le filtre est lavé au dichlorométhane. Le dichlorométhane est enlevé au moyen d'une pompe à vide et l'acide acétique est distillé sous vide en présence d'une trappe pourvue de neige carbonique et d'acétone. Le résidu est repris dans du dichlorométhane, lavé à l'aide d'une solution aqueuse à 10% de carbonate de sodium, est séché sur du carbonate de potassium, est filtré et concentré sous vide pour donner lieu à une poudre blanche (1,231 g (100%).

RMN ($CDCl_3$ δ):
2,4 (large s, 1H), 2,9 (t, 2H), 3,1 (t, 2H), 3,66 (s, 2H), 3,72 (s, 6H), 6,9—7,5 (m, 4H), 8,66 (large s, 1H)
Spectre de masse (m/e):
302 ($M^+$)

Exemple 7

8-méthoxy-1,2,3,4,5,6-hexahydroazépino(4,5-b)indole-5,5-di(carboxylate de méthyle) (VIb)

On agite vigoureusement une solution de 2,16 g (5,12 millimole) de N-benzylamine (Vb) dans 15 ml d'acide acétique sous $H_2$, 1 atmosphère, en présence de 0,21 g de catalyseur à 10% de Pd sur du charbon pendant 3 heures. On sépare le catalyseur par filtration et on lave à l'acide acétique. On enlève le solvant sous vide pour obtenir une huile jaune pâle comme résidu que l'on reprend dans 50 ml de dichlorométhane. On y ajoute un volume égal de $K_2CO_3$ en solution aqueuse saturée et on agite le mélange vigoureusement pendant 10 minutes. On sépare la couche organique et on extrait la couche aqueuse avec 25 ml de dichlorométhane. Les extraits organiques sont rassemblés, séchés sur du carbonate de potassium, filtrés et le solvant est chassé du filtrat pour laisser 1,72 g (100%) d'une mousse blanche que l'on cristallise dans le méthanol de manière à obtenir le produit VIb voulu.

RMN ($CDCl_3$,δ):
8,76 (large s, 1H), 7,4 (d, 1H), 6,8 (m, 2H), 3,8 (s, 3H), 3,78 (s, 6H), 3,7 (s, 2H), 3,14 (m, 2H), 2,90 (m, 2H), 2,34 (s, 1H).
IR (KBr):
3405, 3395, 1755, 1730, 1640, 1600, 1275, 1255, 1235, 1210 $cm^{-1}$
Un échantillon pour analyse, recristallisé dans le méthanol, fond à 156—157°C.
Analyse calc. pour $C_{17}H_{20}N_2O_5$

| | | | |
|---|---|---|---|
| Calc.: | C 61,43% | H 6,07% | N 8,43% |
| Trouvé: | C 61,03% | H 6,15% | N 8,17% |

Exemple 8

Bromure et tétraphénylborate de 3,3[1',5'(2'-éthyl-1'-pentényl)]-1,2,3,4,5,6-hexahydroazépino(4,5-b)-indolinium-5,5-di(carboxylate de méthyle) (VIII X=Br ou tétraphénylborate)

A une solution de 200 mg (0,664 millimole) de dérivé aminodiester de formule VIa dans 8 ml de méthanol sec, tout en agitant et sous atmosphère d'azote, on ajoute 0,194 ml (1,32 millimole) de 5-bromo-2-éthylpentanal VIIa. On agite le mélange 8 heures à 40°C, on le concentre et on reprend le résidu dans du dichlorométhane. L'évaporation du dichlorométhane produit une mousse jaunâtre. On ajoute l'acétate d'éthyle et on casse la mousse en un solide blanchâtre. On filtre cette matière solide et on la lave avec de l'acétate d'éthyle sous une atmosphère d'azote puis on la sèche sous vide pour obtenir 0,225 g (71%) d'une poudre blanchâtre de produit de formule VIII (bromure).

IR (KBr, $\nu_{max}$): 3400, 2950, 1735 (large), 1455, 1435, 1255 (large), 750 $cm^1$
UV (EtOH, $\lambda$ nm):
222, 284, 292 (sh). Comme ce produit solide est hygroscopique et se décompose dans le $CDCl_3$, on prépare le sel de tétraphénylborate en vue de son analyse.

On prépare une solution de 100 mg (0,209 millimole) du spirobromure de formule VIII dans 2 ml de méthanol sec et une solution de 72 mg (0,210 millimole) de tétra- a phénylborate de sodium dans 2 ml de méthanol sec. Chacune de ces solutions est filtrée à travers un tampon de laine de verre et les filtres sont rincés à l'aide de 1 ml de méthanol sec. A la solution de spirobromure (formule VIIa) sous agitation vigoureuse, on ajoute en une fois la solution de tétraphénylborate de sodium. L'addition donne lieu à une solution jaune-clair. Après 10 secondes environ la solution devient trouble et produit un précipité solide. On agite la suspension une demi-heure puis on la filtre et on lave la matière solide à l'aide de méthanol sec, puis on la sèche sous vide pour obtenir 120 mg (80%) d'une poudre blanche de composé voulu (tétraphénylborate VIII). Le produit résiste à toutes les tentatives de cristallisation et se dépose sous forme de solid blanc dans tous les systèmes de solvants utilisés. Le mélange acétone/méthanol donne un produit dont les caractéristiques sont les suivantes:

P.F. 244—247°C
Analyse calc. pour $C_{47}H_{49}N_2O_4B$:
C 78,76% H 6,87% N 3,91%
Trouvé: C 78,93% H 6,59% N 3,69%
IR (KBr, $\nu_{max}$):
3420 (large), 3050, 3000, 2980, 1755 (sh), 1735, 1580, 1475, 1465, 1455 (sh), 1430, 1275, 1220, 775, 760, 740, 735, 710, 610 $cm^{-1}$.

## Exemple 9

Vincadifformine (Ia)

A une solution de 1 g (3,31 millimole) de diesteramine de formule VIa dans 20 ml de méthanol sec, tout en agitant sous une atmosphère d'azote, on ajoute 0,533 ml (3,64 millimole, 0,703 g) de 5-bromo-2-éthylpentanal (cf. W. Oppolzer, H. Hauth, P. Pfaffli, R. Wenger, Helv. *60* 1861 (1977)). On agite la solution une demi-heure à température ambiante et on la chauffe à 40°C, on l'agite 5 heures et on y ajoute ensuite 1 ml de triéthylamine. Après 30 heures d'agitation continue à 40°C, on refroidit la solution et on évapore le solvant, ce qui laisse une huile orange visqueuse.

La chromatographie en colonne de silice et l'élution à l'éther donnent un produit huileux contaminé de 5-bromo-2-éthylpentanal qui n'a pas réagi. En recristallisant dans l'acétonitrile on obtient 0,285 g (26%) de vincadifformine de formule Ia dont les caractéristiques sont les suivantes:

P.F. 124—125°C
IR (KBr, $\nu_{max}$):
3350, 2950, 2930, 2760, 1665, 1600, 1460, 1425, 1325, 1305, 1290, 1275, 1250, 1235, 1225, 1215, 1205, 1185, 1160, 1155, 1045, 755 $cm^{-1}$
RMN ($CDCl_3$, $\delta$):
0,6 (t, 3H), 8,9—3,3 (m, 15H), 3,8 (s, 3H), 6,8—7,36 (m, 4H), 9,02 (large s, 1H)
Spectre de masse (m/e) 338 ($M^+$), 124 (100%).

En variante, on agite le bromure de spiroenammonium VIII 6 heures à 60°C dans le méthanol contenant de la triéthylamine et on obtient de cette manière de la vincadifformine Ia identifiée en comparant ses données spectroscopiques avec celles de l'échantillon obtenu précédemment.

## Exemple 10

11-méthoxyvincadifformine (Ib)

A une solution de 0,45 g (1,4 millimole) de diesteramine VIb et 1 cristal d'acide p-toluènesulfonique dans 30 ml de méthanol sec, on ajoute tout en agitant sous $N_2$, 0,22 ml (0,29 g (1,5 millimole) de bromoaldéhyde VIIa. On continue d'agiter une demi-heure à 20°C et 10 heures à 40°C. A ce moment, une chromatographie en couche mince ($SiO_2$ — 5% de méthanol dans le dichlorométhane) ne montre plus aucun produit de départ. On ajoute à la solution 0,5 ml de triéthylamine, on chauffe la solution à 60°C et on agite 4 heures. On refroidit la solution à 20°C et on enlève le solvant pour obtenir une gomme orange. La chromatographie (colonne de $SiO_2$ avec 5% de méthano dans du dichlorométhane) ne révèle aucune séparation. Pour cette raison, on soumet le produit à une chromatographie préparatoire en couche mince sur des plaques de $SiO_2$ de 2 mm et on développe à l'éther. On recueille les bandes à $R_f$ 0,7, on lave la silice avec 10% de méthanol dans de l'éther, on filtre, on chasse le solvant pour obtenir une mousse blanche: 0,293 g (59%).

RMN ($CDCl_3$, δ):
8,88 (large s, 1H), 7,04 (d, 1H), 6,36 (m, 2H), 3,74 (s, 6H), 3,27—0,69 (m, 14H), 0,55 (t, 3H)
IR (KBr):
3390, 1685, 1620, 1500, 1270 $cm^{-1}$
MS (80eV) m/e:
368 ($M^+$), 124 (base)
UV (éthanol) $\lambda_{max}$ (log ε):
249 (4,00), 330 (4,12).

La chromatographie en couche mince de ce produit ($SiO_2$ — 5% méthanol dans $CH_2Cl_2$) montre une tache correspondant à $R_f$ 0,4 qui se colore en bleu avec le réactif de pulvérisation au sulfate d'ammonium cérique et à l'acide phosphorique.

Le produit cristallise dans le méthanol et fond à 90—92°C Il forme un chlorhydrate à l'état de gomme lorsqu'il est traité avec de l'acide chlorhydrique en solution dans l'éther. En ajoutant soigneusement une solution méthanolique d'acide chlorhydrique à une solution d'amine dans le méthanol jusqu'à atteindre juste le point acide, puis en enlevant le méthanol et en triturant l'huile obtenue de cette façon, on obtient un produit solide amorphe qui résiste aussi à la cristallisation. Un spectre IR de ce produit solide révèle la présence d'une quantité importante d'ester saturé (1730 $cm^{-1}$) qui implique la réaction du système de β-anilino acrylate avec le HCl.

En variante, on forme un picrate cristallin par addition de 53,8 mg d'acide picrique dans la quantité minimum d'éthanol à 72 mg de l'amine Ib également dans la quantité minimum d'éthanol. La recristallisation dans de l'éthanol à 95% donne un échantillon analytique.

P.F. 183—184 (d)°C.
Analyse calc. pour $C_{28}H_{31}N_5O_{10}$

| | | | |
|---|---|---|---|
| Calc.: | C 56,27% | H 5,23% | N 11,72% |
| Trouvé: | C 56,34% | H 5,47% | N 11,90% |

Exemple 11

Synthèse du 2-éthyl-4-oxyranylpentanal VIIc

*a) 2-éthyl-4-oxyranylpentanoate de méthyle*

A du 2-éthyl-4,5-déhydropentanoate de méthyle (3,85 g, 27 millimoles) dans du chlorure de méthylène (25 ml) à 0°C, on ajoute de l'acide m-chloroperbenzoïque (6,5 g, 85%, 32 millimoles). On porte la solution à température ambiante et on l'agite 12 heures. Le solide qui se forme est filtré, lavé au chlorure de méthylène et les solutions rassemblées sont lavées à l'aide d'une solution aqueuse saturée de carbonate de sodium, séchées sur du $MgSO_4$ et concentrées. La distillation (100°C, 25 mm) de l'huile brute donne l'époxyde correspondant (3,5 g, 81%).

IR (net):
2960, 1733, 1192, 1172 $cm^{-1}$
RMN ($CDCl_3$) δ:
0,90 (3H, t), 1,4—2,0 (4H, m), 2,38—2,68 (2H, m), 2,75 (1H, t), 2,95 (1H, m), 3,74 (3H, s).

*b) 2-éthyl-4-oxyranylpentanal VIIc*

L'ester époxy (2,08 g, 13,1 millimoles) est placé dans du chlorure de méthylène (20 ml) sous azote et est refroidi à −78°C. Tout en agitant vigoureusement on y ajoute goutte à goutte en 10 minutes de l'hydrure de diisobutylaluminium (1,2 équivalent, 20% dans l'hexane).

On agite le milieu de réaction pendant 20 autres minutes puis on arrête la réaction brusquement à l'aide de méthanol (2 ml) à −78°C. On verse la solution dans de l'eau et on l'extrait à l'aide de chlorure de méthylène. Les sels d'aluminium formés sont séparés par filtration en utilisant la gravité, à travers de la laine de verre et on les lave au chlorure de méthylène. Les extraits rassemblés sont séchés ($MgSO_4$) et concentrés sous vide. La distillation (60—65°C, 25 mm) fournit le 2-éthyl-4-oxyranylpentanal VIIc (925 mg, 55%).

IR (net):
3055, 3023, 2910, 1720, 1450, 1260 $cm^{-1}$
RMN ($CDCl_3$) δ:
1,0 (3H, t), 1,1—2,1 (4H, m), 2,3—2,6 (2H, m), 2,85 (1H, t), 3,05 (1H, m), 9,85 (1H, d).

Exemple 12

14-hydroxyvincadifformine Ic et 14-hydroxyméthyl-E-norvincadifformine Id

On chauffe au reflux l'azépinoindole VIa (460 mg, 2,1 millimoles) et 0,5 ml de triéthylamine ainsi que l'époxyaldéhyde VIIc (0,5 g, 3,9 millimoles) sous atmosphère d'azote dans le méthanol (10 ml) pendant 8 heures. On enlève le méthanol sous vide. Le composé Id cristallise après une nuit de repos. On le recristallise dans l'acétonitrile.

P.F. 153—154°C (503 mg, 68%). Le résidue est purifié par une chromatographie préparatoire en couche mince (gel de silice, 3% de méthanol dans le chlorure de méthylène, rf 0,6 pour Ic et 0,15 pour Id) donne un deuxième constituant (Ic) sous forme de solide amorphe (105 mg, 14%).

Pour Id: IR (KBr 3260, 2950, 1682, 1605 $cm^{-1}$
UV (MeOH) nm 226, 298, 328
Spectre de masse (m/e) 354 ($M^+$)
Pour Ic: IR (film 3360 (large), 2950, 2790, 1640, 1600 $cm^{-1}$
UV (MeOH) nm 228, 298, 328
Spectre de masse (m/e) 354 ($M^+$)

Exemple 13

Synthèse du 4-(bromométhyl)hexanal VIIb

*a) lactone de l'acide 4-éthyl-5-hydroxypentanoïque*

On refroidit du 4-formyl-hexanoate de méthyle (15,3 g, 97 millimoles) à 0°C dans du méthanol anhydre (125 mg) et on y ajoute du borohydrure de sodium (1,84 g, 48 millimoles) à une vitesse telle que la réaction reste en dessous de 20°C. On l'agite pendant 30 minutes après la fin de l'addition et on verse le tout dans de l'eau. On extrait la solution aqueous avec de l'éther (3 x 75 ml). On rassemble les extraits et on les lave à l'aide d'une saumure saturée, on sèche sur $MgSO_4$ et on concentre sous vide. On reprend l'huile dans du benzène (200 ml) et on y ajoute de l'acide p-toluènesulfonique (1 g). On chauffe la solution au reflux 15 heures en utilisant une trappe de Dean-Stark remplie de chlorure de calcium anhydre. Après refroidissement, on lave le mélange réactionnel au moyen d'une solution saturée de bicarbonate de sodium, on sèche sur $MgSO_4$ et on évapore le solvant. La distillation du produit brut donne la lactone de l'acide 4-éthyl-4-pentanoïque Eb. 70—75°C (0,25 mm) (7,2 g, 58%).

IR (net):
2968, 1730, 1180, 1056 $cm^{-1}$
RMN ($CDCl_3$)$\delta$:
0,98 (3H, t), 1,2—2,2 (5H, m), 2,58 (2H, m), 4,0 (1H, d de d), 4,35 (1H, m).

*b) 4-bromométhyl hexanoate de méthyle*

On fait barboter du HBr gazeux anhydre dans la lactone de l'acide 4-éthyl-5-hydroxypentanoïque (3 g, 23 millimoles) à 60°C pendant 30 minutes. On laisse refroidir la solution et on y ajoute du méthanol (15 ml) avec de l'orthoformiate de triméthyle (2,5 g, 23 millimoles). On agite la solution 8 heures, on la concentre et on la distille Eb 70—75°C (0,1 mm) pour obtenir le bromoester voulu (4,3 g, 83%).

IR (net):
2955, 1733, 1170 $cm^{-1}$
RMN ($CDCl_3$)$\delta$:
0,98 (3H, t), 1,3—2,0 (5H, m), 2,45 (2H, t), 3,6 (2H, d), 3,82 (3H, s).

*c) 4-bromométhylhexanal VIIb*

Le bromoester mentionné ci-dessus (1,9 g, 8,5 millimoles) est dissous dans du chlorure de méthylène anhydre (20 ml) et est refroidi sous forte agitation à −78°C. On y ajoute goutte à goutte de l'hydrure de diisobutylaluminium (10,2 ml, 1 M dans l'hexane) en 10 minutes. On agite la solution pendant 20 minutes supplémentaires à −78°C puis on l'arrête brusquement en y ajoutant du méthanol (2 ml). On verse la solution dans du HCl (3%) on extrait au chlorure de méthylène (3 x 30 ml) et on sèche ($MgSO_4$). L'enlèvement du solvant laisse une huile légère que l'on purifie par distillation Eb 78°C (0,4 mm) (1,38 g, 84%).

IR (net):
2959, 2720, 1720 $cm^{-1}$
RMN ($CDCl_3$)$\delta$:
0,97 (3H, t), 1,3—2,0 (5H, m), 2,57 (2H, t), 3,6 (2H, d), 10,15 (1H, t).

Exemple 14

Pseudovincadifformine Ie (14-éthyl-18, 19-dinorvincadifformine) et épi-14-pseudovincadifformine Ie'

On dissout l'azépinoindole VIa (0,92 g, 4,1 millimoles) dans du méthanol (30 ml) à température ambiante et on y ajoute le bromoaldéhyde VIIb (1.0 g, 5,3 millimoles). On agite la solution 4 heures puis on y ajoute de la triéthylamine (1 ml, excès) et on chauffe la solution à 40°C tout en agitant 16 heures.

On enlève le méthanol sous vide et on reprend le résidu dans du chlorure de méthylène (75 ml), on le lave à l'aide de carbonate de sodium en solution aqueuse saturée, on sèche ($MgSO_4$) et on concentre. Par HPLC (en utilisant une colonne de mciroporacil produit commercial de 10 pouces, soit

25,4 cm, avec un débit de 0,9 ml/minute) en éluant au chloroforme, on obtient deux constituants le (temps de rétention 10,3 minutes) et le' (temps de rétention 8,5 minutes) avec un rapport de 4:1 qui correspond aux deux constituants (rapport 4:1) d'un échantillon de pseudo-vincadifformine naturelle. Une chromatographie en colonne à moyenne pression (4 pieds x 1,25 pouce soit environ 1,20 m x 3,17 cm, gel de silice) en éluant au chloroforme, permet d'isoler le constituant isomère principal comme produit homogène (par HPLC) dont la cristallisation est amorcée en triturant avec un mélange de méthanol et d'eau. Le produit ainsi obtenu est recristallisé dans un mélange de méthanol et d'eau (95:5)

P. F. 118—119°C (155 mg, 11%). L'enrichissement du mélange restant d'épimères (415 mg, 30 %) par cristallisation produit un mélange en proportion non supérieur à 1:1.

Pour le IR (KBr):
3365, 2955, 2773, 1666, 1605, 740 $cm^{-1}$
RMN ($CDCl_3$) $\delta$:
0,95 (3H, t), 1,1—1,6 (4H, m), 1,6—2,15 (4H, m), 2,15—2,65 (3H, m), 2,65—2,95 (4H, m), 3,68 (3H, s), 6,5—6,7 (2H, m), 6,8—7,2 (2H, m), 8,7 (1H, large)
UV (méthanol) nm:
228, 298, 328.

Pour un mélange de le et le' (environ 1:1) le spectre de RMN montre un déplacement des absorptions qui indique un plus grand nombre de protons dans la région $\delta$ 1,6—2,5 et un moins grand nombre dans la région de $\delta$ 1,1—1,6.

Exemple 15

2-benzyl-8-méthoxy-1,2,3,4-tétrahydro-5H-pyrido[4,3-b]indole (IIIc)

En suivant le mode opératoire de préparation de la 7-méthoxytétrahydro-$\gamma$-carboline IIIb tel que décrit à l'exemple 3, mais en utilisant le chlorhydrate de p-méthoxyphénylhydrazine, on prépare l'isomère 8-méthoxy

P.F. 119—120°C avec un rendement de 53%.
RMN ($CDCl_3$)$\delta$:
7,84 (s, 1H), 7,27 (m, 5H), 6,93 (d, J=8Hz, 1H), 6,72 (d, J=2Hz, 1H), 6,65 (d de d, J=8Hz, 2Hz, 1H), 3,74 (s, 5H), 3,62 (s, 2H), 2,67 (m, 4H)
IR (KBr) $\nu_{max}$:
3390, 2930, 2820, 2760, 1595, 1485, 1455, 1220, 1150, 1030, 985, 835, 825, 805, 760, 735, 705, 700 $cm^{-1}$
UV (éthanol) $\lambda_{max}$ 238, 290 m$\mu$
Analyse calc. pour $C_{19}H_{20}N_2O$:

| | | | |
|---|---|---|---|
| | C 78,05 | H 6,90 | N 9,58 |
| Trouvé | C 78,05 | H 6,86 | N 9,47 |

Exemple 16

2-benzyl-8-chloro-1,2,3,4-tétrahydro-5H-pyrido[4,3-b]indole (IIId)

Un précipité lourd se forme par agitation de 2,00 g (11,2 millimoles) de 4-chlorophénylhydrazine et de 2,11 g (11,2 millimoles) de N-benzyl-4-pipéridone pendant 2 heures sous une atmosphère d'azote dans 40 ml d'acide acétique à 22°C. En ajoutant 60 ml de CHl à 8% dans de l'acide acétique, il se forme une solution rouge clair. On la chauffe 2 h. à 75°C en formant ainsi un précipité. Après concentration sous vide à 70°C pour obtenir 40 ml et refroidissement à 22°C, on filtre le précipité et on le rince avec du dichlorométhane. Après suspension du solide résiduel dans 100 ml de dichlorométhane et 100 ml d'eau et addition de carbonate de potassium jusqu'à dissolution de tout le produit solide de départ, on sépare les phases et on extrait la phase aqueuse fortement basique à l'aide de 50 ml de dichlorométhane. Après séchage ($K_2CO_3$), concentration et cristallisation dans de l'éthanol à 95%, on obtient 2,77 g (84%) de tétrahydro-$\gamma$-carboline IIId

P.F. 117—118°C
RMN ($CDCl_3$)$\delta$:
8,12 (s, 1H), 7,29 (m, 6H), 6,69 (s, 2H), 3,74 (s, 2H), 3,60 (s, 2H), 2,70 (m, 4H).

Le composé montre du solvant de cristallisation pour $\delta$ 1,96 que l'on peut enlever par séchage à 60°C (0,05 mm)

IR (KBr) $\nu_{max}$:
3300, 2940, 2840, 2790, 2765, 1590, 1450, 1055, 975, 900, 855, 805, 750, 700 $cm^{-1}$
UV (éthanol) $\lambda_{max}$ 240, 290 nm
Analyse calc. pour $C_{18}H_{17}N_2Cl$

| | | | | |
|---|---|---|---|---|
| | C 72,84 | H 5,77 | N 9,44 | Cl 11,95 |
| Trouvé | C 72,70 | H 5,87 | N 9,38 | Cl 12,11 |

Exemple 17

2-benzyl-8-bromo-1,2,3,4-tétrahydro-5H-pyrido[4,3-b]indole IIIe

En suivant le mode opératoire d'obtention de la 8-chlorotétrahydro-$\gamma$-carboline IIId, mais en utilisant la 4-bromophénylhydrazine sous forme de chlorhydrate, on forme l'isomère 8-bromo IIIe

P.F. 122—123°C avec un rendement de 86%
RMN ($CDCl_3$) $\varepsilon$:
8,18 (s, 1H), 7,42 (d, J=2Hz, 1H), 7,32 (s, 5H), 7,07 (d de d, $J_{H7-H9}$=2Hz, $J_{H7-H6}$=8Hz, 1H), 6,86 (d, J=8Hz, 1H), 3,72 (s, 2H), 3,58 (s, 2H), 2,66 (m, 4H)
IR (KBr) $\nu_{max}$:
3400, 2905, 2890, 2800, 2780, 1570, 1440, 1050, 980, 790, 740, 695 $cm^{-1}$
UV (éthanol) $\lambda_{max}$ 240, 290 nm
Analyse calc. pour $C_{18}H_{17}N_2Br$:

| | | | | |
|---|---|---|---|---|
| | C 63,35 | H 5,02 | N 8,21 | Br 23,42 |
| Trouvé | C 63,25 | H 5,18 | N 8,18 | Br 23,69 |

Exemple 18

3-benzyl-9-méthoxy-1,2,3,4,5,6-hexahydroazépino[4,5-b]indole-5,5-di(carboxylate de méthyle) Vc

En suivant le mode opératoire de préparation de la 8-méthoxyindoloazépine Vb tel que décrit à l'exemple 5, on prépare l'isomère 9-méthoxy Vc, P.F. 128—130°C avec un rendement de 69%.

RMN ($CDCl_3$) $\delta$:
8,34 (large s, 1H), 7,29 (m, 6H), 6,90 (m, 2H), 3,81 (s, 3H), 3,76 (s, 6H), 3,67 (s, 2H), 2,87 (s, 4H)
IR (KBr) $\nu_{max}$:
3400, 2960, 2840, 1749, 1722, 1480, 1465, 1450, 1430, 1250, 1140, 1030, 835, 740, 700 $cm^{-1}$
Analyse calc. pour $C_{24}H_{26}N_2O_5$

| | | | |
|---|---|---|---|
| | C 68,33 | H 6,20 | N 6,63 |
| Trouvé | C 68,26 | H 6,24 | N 6,47 |

Exemple 19

3-benzyl-9-chloro-1,2,3,4,5,6-hexahydroazépino[4,5-b]indole-5,5-di(carboxylate de méthyle) Vd

En suivant le mode opératoire de préparation de la 9-méthoxy indoloazépine Vc, on prépare le produit 9-chloro Vd.

P.F. 111—113°C avec un rendement de 60%.
RMN ($CDCl_3$) $\delta$:
8,49 (s, 1H), 7,22 (m, 8H), 3,78 (s, 2H), 3,74 (s, 6H), 3,65 (s, 2H), 2,84 (s, 4H)
IR (KBr) $\nu_{max}$:
3404, 2960, 2920, 2890, 2840, 1725, 1455, 1430, 1320, 1260, 1250, 1230, 1130, 1050, 880, 803, 802, 740, 735, 695 $cm^{-1}$
UV (éthanol) $\lambda_{max}$ 239, 295 nm.
Analyse calc. pour $C_{23}H_{23}N_2O_4Cl$

| | | | | |
|---|---|---|---|---|
| | C 64,71 | H 5,43 | N 6,56 | Cl 8,31 |
| Trouvé | C 64,67 | H 5,55 | N 6,52 | Cl 8,59 |

Exemple 20

3-benzyl-9-bromo-1,2,3,4,5,6-hexahydroazépino[4,5-b]indole-5,5-di(carboxylate de méthyle) Ve

En suivant le mode opératoire de préparation de la 9-méthoxy indoloazépine Vc, on prépare le produit 9-bromo Ve

P.F. 116—118°C avec un rendement de 60%

RMN ($CDCl_3$) $\delta$:

8,48 (large s, 1H), 7,54 (s, 1H), 7,25 (s, 5H), 7,14 (s, 2H), 3,77 (s, 2H), 3,72 (s, 6H), 3,62 (s, 2H), 2,81 (s, 4H)

IR (KBr) $\nu_{max}$:

3440, 2950, 2905, 2815, 1745, 1732, 1465, 1435, 1245, 1220, 1140, 1045, 1025, 790, 740, 695 $cm^{-1}$

UV (éthanol) $\lambda_{max}$ 238, 293 nm

Analyse calc. pour $C_{23}H_{23}N_2O_4Br$

| | | | | |
|---|---|---|---|---|
| | C 58,60 | H 4,92 | N 5,94 | Br 16,95 |
| Trouvé | C 58,52 | H 4,86 | N 5,72 | Br 17,11 |

Exemple 21

9-méthoxy-1,2,3,4,5,6-hexahydroazépino[4,5-b]indole-5,5-di(carboxylate de méthyle) VIc

En effectuant une débenzylation de la 9-méthoxyazépinoindole Vc selon le mode opéreatoire donné pour le composé parent VIa à l'exemple 6 et en cristallisant dans le méthanol, on obtient l'amine secondaire VIc sous forme de méthanolate avec un rendement quantitatif

P.F. 95—105°C

RMN ($CDCl_3$) $\delta$:

8,75 (large s, 1H), 7,10 (d, J=8Hz, 1H), 6,99 (d, J=2Hz, 1H), 6,77 (d de d, J=8Hz, 2Hz, 2H), 3,82 (s, 3H), 3,74 (s, 8H), 3,03 (m, 4H), 2,38 (large s, 1H)

IR (KBr) $\nu_{max}$:

3400, 3300, 2950, 2840, 1740, 1720, 1215, 1050 $cm^{-1}$

Analyse calc. pour $C_{18}H_{24}N_2O_6$

| | | | |
|---|---|---|---|
| | C 59,33 | H 6,64 | N 7,69 |
| Trouvé | C 59,33 | H 6,68 | N 7,66 |

Exemple 22

9-chloro-(et 9-bromo)-1,2,3,4,5,6-hexahydroazépino[4,5-b]indole-5,5-di(carboxylate de méthyle) (VId et VIe)

Pour éviter une déschalogénation importante que l'on avait constatée en appliquant la débenzylation par réduction selon le mode opératoire relatif au composé parent Va, on effectue l'hydrogénation des dérivés chloro et bromo Vd et Ve dans le méthanol contenant 3% de HCl anhydre. On arrête l'hydrogénation après absorption de 1,05 équivalent d'hydrogène.

La chloroamine VId est obtenue proprement mais on ne peut pas éviter une certaine déshalogénation du composé bromo.

Le chloroazépinoindole VId est donc obtenu avec un rendement quantitatif en recristallisant dans un mélange d'acétate de méthyle et d'hexane.

RMN ($CDCl_3$) $\delta$:

8,86 (large s, 1H), 7,44 (large s, 1H), 7,14 (m, 2H), 3,77 (s, 6H), 3,68 (s, 2H), 2,99 (m, 4H), 2,28 (large s, 1H)

IR (KBr) $\nu_{max}$:

3370, 3350, 2930, 2830, 1735, 1715, 1463, 1435, 1260, 1240, 1215, 800 $cm^{-1}$

Spectre de masse (80eV) m/e: 336 ($M^+$)

Analyse calc. pour $C_{16}H_{17}N_2O_4Cl$

| | | | | |
|---|---|---|---|---|
| | C 57,06 | H 5,09 | N 8,32 | Cl 10,53 |
| Trouvé | C 56,96 | H 5,34 | N 8,09 | Cl 10,43 |

Le bromoazépinoindole VIe est obtenu avec une pureté d'environ 80%. Le composé est contaminé par le produit déshalogéné VIa. Comme on le voit par chromatographie en couche mince et spectre de RMN qui montrent des signaux NH respectifs à $\delta$ 8,98 et 8,86 (conc. dépendant dans $CDCl_3$) pour IVe et VIa. Les difficultés de séparation et de purification de VIe encouragent l'utilisation du produit brut dans l'étape de synthèse subséquente.

RMN ($CDCl_3$) $\delta$:

8,86 (large s, 1H), 7,59 (s, 1H), 7,18 (large s, 2H), 3,76 (s, 6H), 3,66 (s, 2H), 2,99 (m, 4H), 2,26 (large s, 1H).

Spectre de masse (80eV) 380 ($M^+$)

Exemple 23

10-chloro, 10-bromo et 10-méthoxyvincadifformine (If,g,h)

On prépare ces composés en suivant le mode opératoire donné pour la vincadifformine Ia. On purifie les produits chloro et bromo par chromatographie préparatoire en couche mince sur de la silice Merck, on développe au moyen de méthanol à 2% dans du dichlorométhane.

On sépare aisément la bromovincadifformine Ig Rf 0,60 du constituant mineur, la vincadifformine Ia Rf 0,48 formé par l'impureté indoloazépine VIa.

Le composé chloro If (rendement 70%) cristallise dans le méthanol,

P.F. 131—132°C.
RMN ($CCl_4$):
8,98 (large s, 1H), 702 (m, 2H), 6,71 (d, J=8Hz, 1H), 3,72 (s, 3H), 3,20—0,80 (large m, 15H), 0,64 (+, 3H).
IR ($CHCl_3$) $\nu_{max}$:
3370, 2930, 2770, 1665, 1600, 1465, 1290, 1260, 1150 $cm^{-1}$
UV (méthanol) $\lambda_{max}$ (log $\varepsilon$) 225 (s, 4,11), 331 (4, 20), 338 (4,17)
Spectre de masse (80eV) m/e (% rel) 372 (45), 125 (15), 124 (100)
Analyse calc. pour $C_{21}H_{25}N_2O_2Cl$

| | | | | |
|---|---|---|---|---|
| | C 67,64 | H 6,76 | N 7,51 | Cl 9,51 |
| Trouvé | C 67,67 | H 6,75 | N 7,41 | Cl 9,77 |

Le composé bromo Ig (rendement 51%) résiste à la cristallisation.

RMN ($CCl_4$) $\delta$:
8,94 (large s, 1H), 7,10 (m, 2H), 6,58 (d, J=10Hz, 1H), 3,64 (s, 3H), 3,20—0,76 (large m, 15H), 0,60 (t, 3H)
IR ($CHCl_3$) $\nu_{max}$:
3360, 2930, 2775, 1665, 1460, 1290, 1260, 1150 $cm^{-1}$
UV (méthanol) $\lambda$ (log $\varepsilon$) 225 (sh; 4,11), 310 (4, 19), 331 (4,17)

Spectre de masse (80eV) m/e (% rel) 418 (12), 416 (12), 125 (39), 124 (100).

Le composé forme un picrate qui cristallise dans l'éthanol à 95%,

P.F. 203—204°C.
Analyse calc. pour $C_{27}H_{28}N_5O_9Br$

| | | | |
|---|---|---|---|
| | C 50,16 | H 4,37 | N 10,83 |
| Trouvé | C 49,92 | H 4,43 | N 10,64 |

Le composé méthoxy Ih (rendement 80%) est amorphe.

RMN ($CDCl_3$) $\delta$:
8,75 (large s, 1H), 6,77 (large s, 1H), 6,62 (m, 2H), 3,73 (s, 3H), 3,71 (s, 3H), 3,38—0,80 (m, 15H), 0,58 (t, 3H)
IR ($CHCl_3$) $\nu_{max}$:
3380, 2925, 2760, 1660, 1600, 1210, 1140 $cm^{-1}$
UV $\lambda_{max}$ (log $\varepsilon$) 228 (sh; 4,10), 3,13 (4,23), 331 (4,14)
Spectre de masse (80eV) m/e: 368 ($M^+$), 124 (base).

Un picrate est recristallisé dans de l'éthanol à 95%,

P.F. 152—153°C
Analyse calc. pour $C_{28}H_{31}N_5O_{10}$

| | | | |
|---|---|---|---|
| | C 56,27 | H 5,23 | N 11,72 |
| Trouvé | C 56,30 | H 5,25 | N 11,66 |

**Revendications**

1. Procédé de préparation de vincadifformine ou d'un composé apparanté à celle-ci, de formule

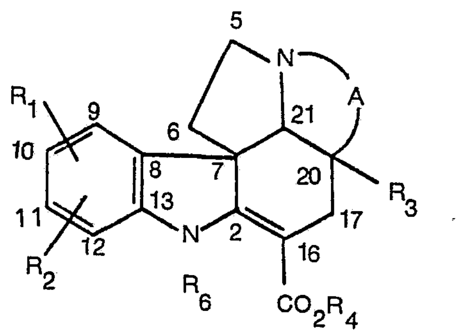

(I)

dans laquelle $R_1$ et $R_2$ désignent de l'hydrogène ou un groupe hydroxy, acyloxy, carbamate, alkoxy, alkyle ou halogéno; ou une combinaison de ces substituants; $R_3$, $R_4$ et $R_6$ sont égaux ou différents et désignent de l'hydrogène ou un groupe alkyle de 1 à 7 atomes de carbone; A représente une chaîne alkyle contenant 2 ou 3 atomes de carbone portant éventuellement un ou plusieurs substituants hydroxy, alkyle ou hydroxyalkyle dont la chaîne contient 1 à 7 atomes de carbone, caractérisé par les opérations suivante de

— transformation d'une N-benzyl-tétrahydro-$\gamma$-carboline (III) de formule

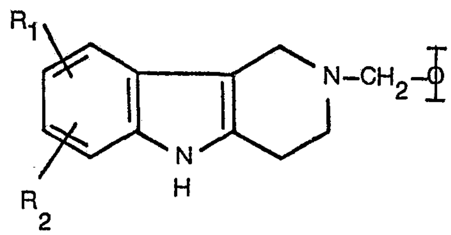

(III)

dans laquelle Φ désigne phényle,
en un 1,2,3,4,5,6-hexahydroazépino(4,5-b)indole-5,5-di(carboxylate d'alkyle) de formule

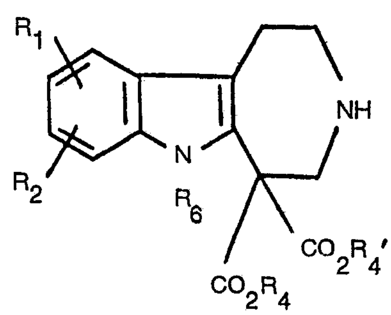

(VI)

dans laquelle $R_1$, $R_2$ et $R_4$ sont tels que définis ci-dessus, $R_4'$ a la même définition que $R_4$ et $R_6$ est H ou alkyle inférieur

— condensation de composé (VI) avec un aldéhyde halogéné, arylsulfonate, alkylsulfonate ou époxyde comportant de 4 à 12 atomes de carbone et dont un dérivé énamine tertiaire peut subir une N-alkylation intramoléculaire pour former la vincadifformine ou un autre composé de formule I apparenté à la vincadifformine.

2. Procédé suivant la revendication 1, caractérisé en ce que la transformation du composé (III) en composé (VI) implique une réaction de la N-benzyl-tétrahydro-$\gamma$-carboline (III) avec l'hypochloride de t-butyle ou un agent d'halogénation similaire pour obtenir une halogénoindolénine que l'on traite directement avec un dérivé métallique de malonate de dialkyle pour former un 3-benzyl-1,2,3,4,5,6-hexahydroazépino(4,5-b)indole-5,5-di(carboxylate d'alkyle) (V) de formule

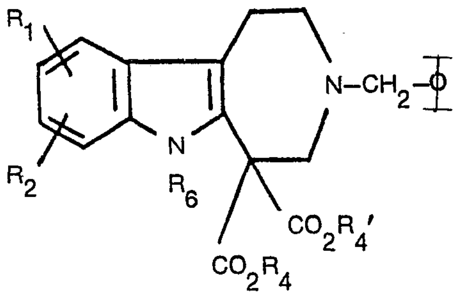

(V)

éventuellement une N-alkylation du composé V où $R_6$ est H pour obtenir le composé V où $R_6$ est alkyle inférieur puis l'hydrogénation du composé (V) en présence de palladium sur charbon ou d'un catalyseur semblable pour obtenir le composé VI.

3. Procédé de préparation de vincadifformine ou d'un composé apparanté de formule I, caractérisé en ce qu'on effectue la condensation d'un 1,2,3,4,5,6-hexahydroazépino(4,5-b)indole-5,5-di(carboxylate d'alkyle) de formule

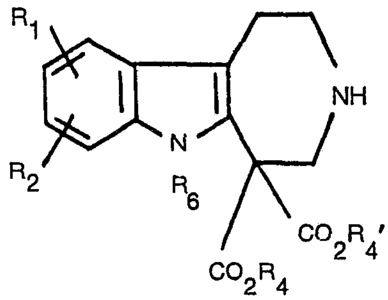

(VI)

dans laquelle $R_1$, $R_2$ et $R_4$ sont tels que définis ci-dessus, $R_4'$ a la même définition que $R_4$; et $R_6$ est H ou alkyle inférieur, avec un aldéhyde fonctionnalisé typiquement un halogéno ou un époxy aldéhyde ramifié ou non, contenant 4 à 12 atomes du carbone.

4. Procédé suivant l'une quelconque des revendications 1 et 2, caractérisé en ce que la N-benzyl-tétrahydro-$\gamma$-carboline (III) est obtenue à partir d'une N-benzyl-pipéridone (IIa)

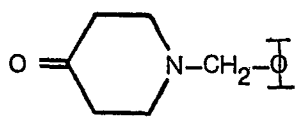

(IIa)

5. Procédé suivant la revendication 4, caractérisé en ce que la N-benzyl-pipéridone (IIa) est mise en réaction avec une phénylhydrazine appropriée pour former une N-benzylpipéridone-phénylhydrazone (IIb) de formule

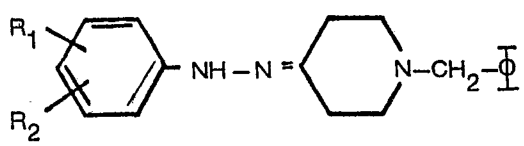

(IIb)

et ce composé-ci est mis en réaction dans les conditions de la synthèse indolique deFisher pour former le composé (III).

6. Procédé de préparation d'un composé intermédiaire de formule

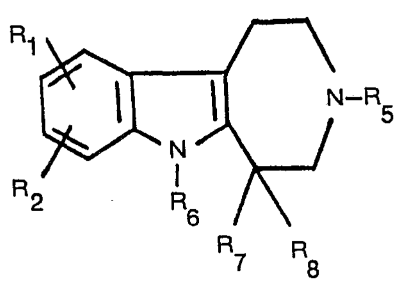

dans laquelle $R_1$ et $R_2$ représentent un atome d'hydrogène, ou un groupe hydroxy, acyloxy, carbamate, alkoxy, alkyle ou halogéno ou une combinaison de ces substituants; $R_5$ représente un atome d'hydrogène ou un groupe benzyle; $R_6$ représente un atome d'hydrogène ou un groupe alkyle comportant 1 à 7 atomes de carbone; $R_7$ et $R_8$ représentent chacun un groupe carbalkoxy dont la partie alkoxy a 1 à 4 atomes de carbone, en pouvant être différents l'un de l'autre, caractérisé en ce qu'on fait réagir une N-benzyl-tétrahydro-$\gamma$-carboline (III)

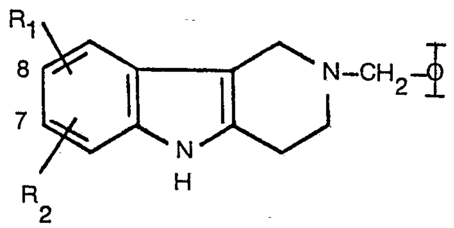

(III)

avec l'hypochlorite de t-butyle ou un agent d'halogénation similaire pour obtenir une halogénoindolénine que l'on traite directement avec un dérivé métallique de malonate de dialkyle pour former un 3-benzyl-1,2,3,4,5,6-hexahydroazépino(4,5-b)indole-5,5-di(carboxylate d'alkyle) (V) c'est-à-dire un composé répondant à la formule générale donnée ci-dessus où $R_5$ représente un groupe benzyle, $R_6$ représente un atome d'hydrogène et $R_7$ et $R_8$ représentent l'un et l'autre un groupe carbalkoxy, et le composé ainsi obtenu peut être utilisé tel quel ou, si on le préfère, transformé en un autre composé de ladite formule générale, par l'un ou l'autre des traitements suivants:

— débenzylation par hydrogénation en présence de palladium sur charbon ou d'un composé similaire pour obtenir un 1,2,3,4,5,6-hexahydroazépino (4,5-b)indole-5,5-di(carboxylate d'alkyle) (VI) c'est-à-dire un composé de ladite formule générale où $R_5$ représente un atome d'hydrogène, $R_6$ représente un atome d'hydrogène et $R_7$ et $R_8$ représentent l'un et l'autre un groupe carbalkoxy et/ou

— alkylation pour obtenir un composé de ladite formule générale où $R_6$ représente un groupe alkyle.

7. En tant que nouveaux composés intermédiaires ceux de formule

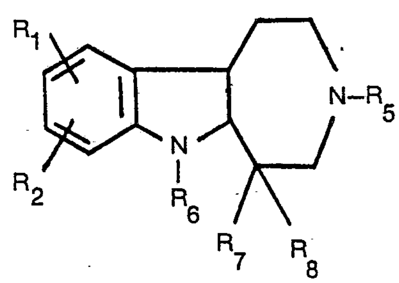

dans laquelle $R_1$ et $R_2$ représentent un atome d'hydrogène, ou un groupe hydroxy, acyloxy, carbamate, alkoxy, alkyle ou halogéno ou une combinaison de ces substituants; $R_5$ représente un atome d'hydrogène ou un groupe benzyle; $R_6$ représente un atome d'hydrogène ou un groupe alkyle comportant 1 à 7 atomes de carbone; $R_7$ et $R_8$ représentent chacun un groupe carbalkoxy dont la partie alkoxy comporte 1 à 4 atomes de carbone et qui peuvent différer l'un de l'autre, à l'exception des composés dans lesquels $R_1$, $R_2$ et $R_6$ représentent chacun un atome d'hydrogène, $R_5$ représente un groupe benzyle ou un atome d'hydrogène, $R_7$ représente un groupe carboxylate de méthyle et $R_8$ représente un groupe carboxylate de t-butyle.

## Claims

1. Process for preparing vincadifformine or a compound related thereto, of the formula

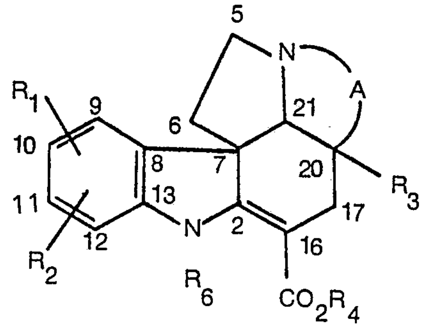

(I)

wherein $R_1$ and $R_2$ refer to hydrogen or a hydroxy, acyloxy, carbamate, alkoxy, alkyl or halo group or a combination of such substituents; $R_3$, $R_4$ and $R_6$ are the same or different and refer to hydrogen or a alkyl group having from 1 to 7 carbon atoms; A represents an alkyl chain containing 2 or 3 carbon atoms possibly carrying one or more hydroxy, alkyl or hyroxyalkyl substituents, the chain of which contains 1 to 7 carbon atoms, characterized by the following steps of

— transformation of a N-benzyl-tetrahydro-$\gamma$-carboline (III) of the formula

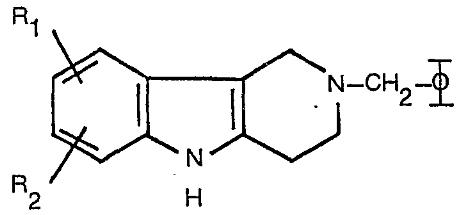

(III)

wherein Φ represents phenyl into a dialkyl 1,2,3,4,5,6-hexahydroazepino(4,5b)indole-5,5-dicarboxylate of the formula

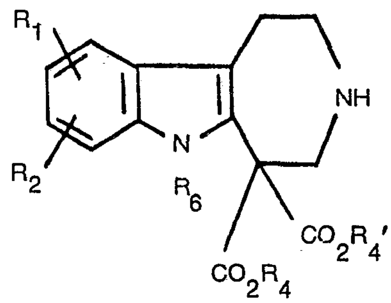

(VI)

wherein $R_1$, $R_2$ and $R_4$ are as defined hereabove, $R_4'$ has the same meaning as $R_4$ and $R_6$ is H or a lower alkyl

— condensation of the compound (VI) with a halogenated aldehyde, an arylsulfonate aldehyde, an alkylsulfonate aldehyde or an epoxy aldehyde of form 4 to 12 carbon atoms and a tertiary enamine derivative of which may undergo an intramolecular N-alkylation to form the vincadifformine or another compound of formula I related to vincadifformine.

2. Process according to claim 1, characterized in that the transformation of compound (III) to compound (VI) involves reacting the N-benzyl-tetrahydro-$\gamma$-carboline (III) with t-butyl hypochlorite or a similar halogenating agent to obtain a haloindolenine which is directly treated with a metal derivative of dialkyl malonate to form a dialkyl 3-benzyl-1,2,3,4,5,6-hexahydroazepino(4,5-b) indôle-5,5 dicarboxylate (V) of the formula

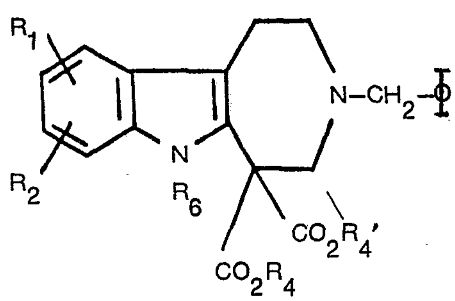

(V)

possibly N-alkylating compound V wherein $R_6$ is H to obtain compound V wherein $R_6$ is lower alkyl and then hydrogenating said compound (V) in the presence of palladium of charcoal or a similar catalyst to obtain compound VI.

3. Process for preparing vincadifformine or a related compound of the formula I, characterized by effecting the condensation of a dialkyl 1,2,3,4,5,6-hexahydroazepino(4,5-b)indole-5,5-dicarboxylate of the formula

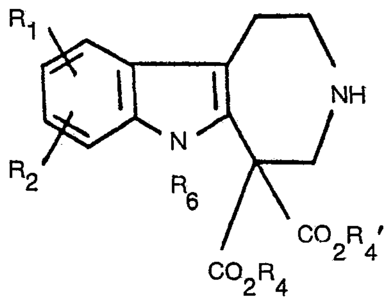

(VI)

wherein $R_1$, $R_2$ and $R_4$ are as defined hereabove, $R_4'$ has the same meaning as $R_4$ and $R_6$ is H or lower alkyl, with a functionalized aldehyde, typically a halo- or epoxy-aldehyde branched or not branched, containing 4 to 12 carbon atoms.

4. Process according to any of claims 1 and 2, characterized by the fact that the N-benzyl-tetrahydro-$\gamma$-carboline (III) is obtained from a N-benzyl-piperidone (IIa)

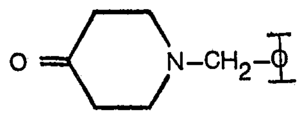

(IIa)

5. Process according to claim 4, characterized in that the N-benzyl-piperidone (IIa) is reacted with an appropriate phenylhydrazine to form a N-benzylpiperidonephenyl-hydrazone (IIb) of the formula

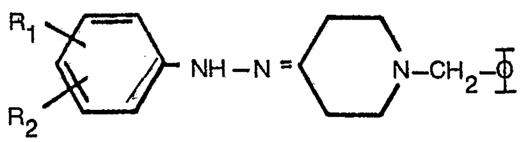

(IIb)

and said compound is caused to react under the conditions of the Fisher indole synthesis so as to form compound (III).

6. Process for preparing an intermediate compound of the formula

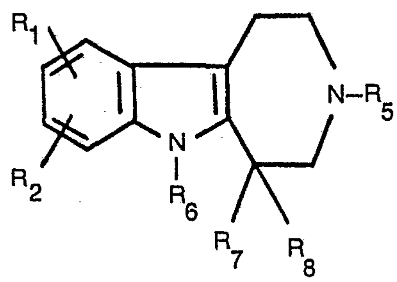

wherein $R_1$ and $R_2$ represent a hydrogen atom or a hydroxy, acyloxy, carbamate, alkoxy, alkyl or halo group or a combination of such substituents; $R_5$ represents a hydrogen atom or a benzyl group; $R_6$ represents a hydrogen atom or an alkyl group having 1 to 7 atoms of carbon; $R_7$ and $R_8$ each represent a carbalkoxy group the alkoxy portion of which has 1 to 4 carbon atoms, and which may be different from one another, characterized by reacting a N-benzyl-tetrahydro-$\gamma$-carboline (III)

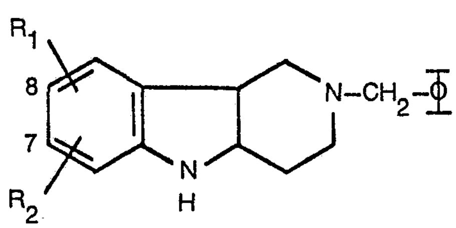

(III)

with t-butyl hypochlorite or a similar halogenating agent to obtain a haloindolenine which is directly treated with a metal derivative of dialkyl malonate to form a dialkyl 3-benzyl-1,2,3,4,5,6-hexahydroazepino(4,5-b)indole-5,5-dicarboxylate (V) namely a compound of the above given general

formula wherein $R_5$ represents a benzyl group, $R_6$ represents a hydrogen atom and $R_7$ and $R_8$ both represent a carbalkoxy group, and the compound thus obtained may be used either as such or, if preferred, may be transformed into another compound of said general formula by anyone of the following steps of:
— debenzylation by hydrogenation in the presence of palladium on charcoal or a similar compound to obtain a dialkyl 1,2,3,4,5,6-hexahydroazepino(4,5-b)indole-5,5-dicarboxylate (VI) namely a compound of said general formula wherein $R_5$ represents a hydrogen atom, $R_6$ represents a hydrogen atom and $R_7$ and $R_8$ both represent a carbalkoxy group and/or
— alkylation in order to obtain a compound of said general formula wherein $R_6$ represents an alkyl group.

7. As new intermediate compounds those of the formula

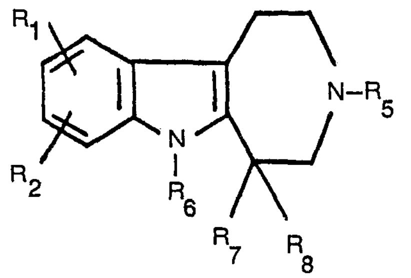

wherein $R_1$ and $R_2$ represent a hydrogen atom or a hydroxy, acyloxy, carbamate, alkoxy, alkyl or halo group or a combination of such substituents; $R_5$ represents a hydrogen atom or a benzyl group; $R_6$ represents a hydrogen atom or an alkyl group having 1 to 7 carbon atoms; $R_7$ and $R_8$ each represent a carbalkoxy group the alkoxy portion of which has 1 to 4 carbon atoms and which may be different from one another, except for those compounds in which $R_1$, $R_2$ and $R_6$ each represent a hydrogen atom, $R_5$ represent a benzyl group or a hydrogen atom, $R_7$ represents a methyl carboxylate group and $R_8$ represents a t-butyl carboxylate group.

**Patentansprüche**

1. Verfahren zur Herstellung von Vincadifformin oder einer damit verwandten Verbindung der Formel

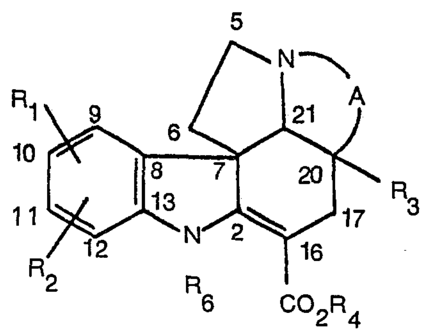

(I)

in der $R_1$ und $R_2$ Wasserstoffatome oder Hydroxylgruppen, Acyloxygruppen, Carbamatgruppen, Alkoxygruppen, Alkylgruppen oder Halogenatome oder eine Kombination dieser Substituenten darstellen; $R_3$, $R_4$ und $R_6$ gleichartig oder verschiedenen sind und Wasserstoffatome oder Alkylgruppen mit 1 bis 7 Kohlenstoffatomen bedeuten; A eine Alkylkette mit 2 oder 3 Kohlenstoffatomen darstellt, die gegebenenfalls einen oder mehrere Hydroxy-, Alkyl- oder Hydroxyalkyl-Substituenten, dessen Kette 1 bis 7 Kohlenstoffatome aufweist, trägt, gekennzeichnet durch die folgenden Maßnahmen
— der Umwandlung eines N-Benzyl-tetrahydro-$\gamma$-carbolins (III) der Formel

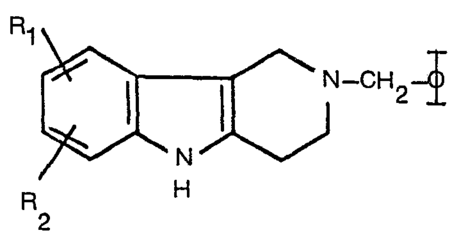

(III)

in der Φ für Phenyl steht, in ein 1,2,3,4,5,6-Hexahydroazepino(4,5-b)indol-5,5-di(alkylcarboxylat) der Formel

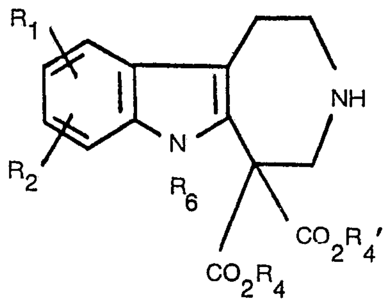

(VI)

in der $R_1$, $R_2$ und $R_4$ die oben angegebenen Bedeutungen besitzen, $R_4'$ die gleichen Bedeutungen besitzt wie $R_4$ und $R_6$ H oder eine niedrigmolekulare Alkylgruppe darstellt,

— der Kondensation der Verbindung der Formel (VI) mit einem halogenierten Aldehyd, einem Arylsulfonataldehyd, einem Alkylsulfonataldehyd oder einem Epoxialdehyd mit 4 bis 12 Kohlenstoffatomen, dessen tertiäres Enaminderivat einer intramolekularen N-Alkylierung unterwofen werden kann, zur Bildung von Vincadifformin oder einer anderen mit Vincadifformin verwandten Verbindung der Formel I.

2. Verfahren nach Anspruch 1, dadurch gekennzeichnet, daß die Umwandlung der Verbindung (III) in die Verbindung (VI) eine Umsetzung von N-Benzyl-tetrahydro-$\gamma$-carbolin (III) mit tert.-Butyl-hypochlorit oder eines ähnlichen Halogenierungsmittels zur Bildung eines Halogenoindolenins umfaßt, welches man direkt mit einem Metallderivat eines Dialkylmalonats unter Bildung eines 3-Benzyl-1,2,3,4,5,6-hexahydroazepino(4,5-b)indol-5,5-di(alkylcarboxylats) (V) der Formel

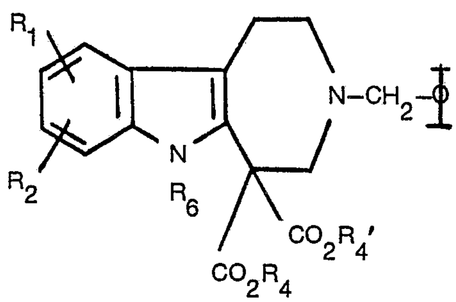

(V)

behandelt, worauf man gegebenenfalls eine N-Alkylierung der Verbindung (V), in der $R_6$ H bedeutet, zur Bildung der Verbindung (V), in der $R_6$ eine niedrigmolekulare Alkylgruppe darstellt, durchführt, worauf man die Verbindung (V) in Gegenwart von Palladium auf Kohlenstoff oder eines ähnlichen Katalysators zur Herstellung der Verbindung (VI) hydriert.

3. Verfahren zur Herstellung von Vincadifformin oder einer damit verwandten Verbindung der Formel I, dadurch gekennzeichnet, daß man die Kondensation eines 1,2,3,4,5,6-Hexahydroazepino(4,5-b)-indol-5,5-di(alkylcarboxylats) der Formel

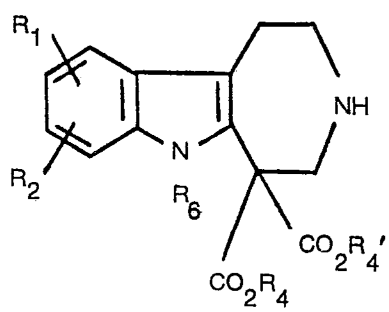

(VI)

in der $R_1$, $R_2$ und $R_4$ die oben angegebenen Bedeutungen besitzen, $R_4'$ die gleichen bedeutungen besitzt wie $R_4$ und $R_6$ H oder eine niedrigmolekulare Alkylgruppe darstellt, mit einem funktionalisierten Aldehyd, typischerweise einem verzweigten oder nicht verzweigten Halogenaldehyd oder Epoxyaldehyd mit 4 bis 12 Kohlenstoffatomen bewirkt.

4. Verfahren nach einem der Ansprüche 1 und 2, dadurch gekennzeichnet, daß N-Benzyl-tetrahydro-$\gamma$-carbolin (III) ausgehend von einem N-Benzyl-piperidon (IIa)

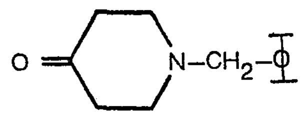

(IIa)

erhalten wird.

5. Verfahren nach Anspruch 4, dadurch gekennzeichnet, daß man das N-Benzyl-piperidon (IIa) mit einem geeigneten Phenylhydrazin zur Bildung eines N-Benzyl-piperidonphenylhydrazons (IIb) der Formel

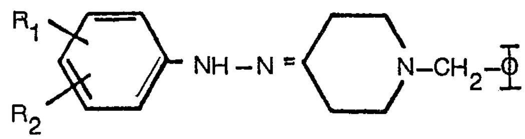

(IIb)

umsetzt und diese Verbindung unter den Bedingungen der Indolsynthese nach Fisher zur Bildung der Verbindung (III) umsetzt.

6. Verfahren zur Herstellung eines Zwischenprodukts der Formel

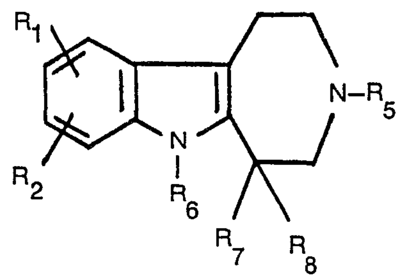

in der $R_1$ und $R_2$ Wasserstoffatome, Hydroxylgruppen, Acyloxygruppen, Carbamatgruppen, Alkoxygruppen, Alkylgruppen oder Halogenatome oder eine Kombination dieser Substituenten darstellen; $R_5$ ein Wasserstoffatom oder eine Benzylgruppe bedeutet; $R_6$ ein Wasserstoffatom oder eine Alkylgruppe mit 1 bis 7 Kohlenstoffatomen darstellt; $R_7$ und $R_8$ jeweils eine Carbalkoxygruppe bedeuten, deren Alkoxyrest 1 bis 4 Kohlenstoffatome aufweist und die voneinander verschieden sein können, dadurch gekennzeichnet, daß man ein N-Benzyl-tetrahydro-$\gamma$-carbolin (III)

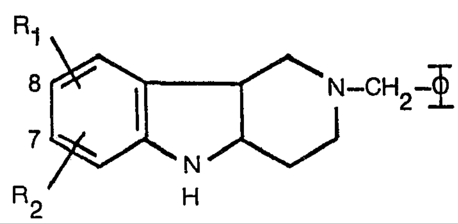

(III)

mit tert.-Butyl-hypochlorit oder einem ähnlichen Halogenierungsmittel umsetzt zur Bildung eines Halogenoindolenins, welches man direkt mit einem Metallderivat eines Dialkylmalonats zur Bildung eines 3-Benzyl-1,2,3,4,5,6-hexahydroazepino(4,5-b)indol-5,5-di(alkylcarboxylats) (V), d.h. einer Verbindung der oben angegebenen allgemeinen Formel, in der $R_5$ eine Benzylgruppe, $R_6$ eine Wasserstoffatom und $R_7$ und $R_8$ jeweils eine Carbalkoxygruppe bedeuten, behandelt und die in dieser Weise herhaltene Verbindung so, wie sie ist, oder gewünschtenfalls nach der Umwandlung in eine andere Verbindung der allgemeinen Formel durch die eine oder die andere der folgenden Behandlungen verwendet:

— Debenzylierung durch Hydrierung in Gegenwart von Palladium auf Kohlenstoff oder einer ähnlichen Verbindung zur Bildung eines 1,2,3,4,5,6-Hexahydroazepino(4,5-b)indol-5,5-di(alkylcarboxylats) (VI), d.h. einer Verbindung der allgemeinen Formel I, in der $R_5$ eine Wasserstoffatom, $R_6$ eine Wasserstoffatom und $R_7$ und $R_8$ jeweils eine Carbalkoxygruppe bedeuten, und/oder

— Alkylierung zur Bildung einer Verbindung der allgemeinen Formel, in der $R_6$ eine Alkylgruppe darstellt.

7. Neue Zwischenprodukte der Formel

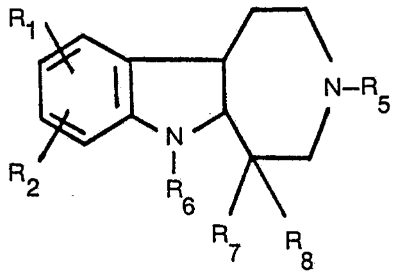

in der $R_1$ und $R_2$ Wasserstoffatome, Hydroxylgruppen, Acyloxygruppen, Carbamatgruppen, Alkoxygruppen, Alkylgruppen oder Halogenatome oder eine Kombination dieser Substituenten darstellen; $R_5$ ein Wasserstoffatom oder eine Benzylgruppe bedeutet; $R_6$ eine Wasserstoffatom oder eine Alkylgruppe mit 1 bis 7 Kohlenstoffatomen darstellt; $R_7$ und $R_8$ jeweils für eine Carbalkoxygruppe stehen, deren Alkoxyrest 1 bis 4 Kohlenstoffatome aufweist, und die von einander verschiedenen sein können, mit Ausnahme der Verbindungen, in denen $R_1$, $R_2$ und $R_6$ jeweils ein Wasserstoffatom, $R_5$ ein Benzylgruppe oder ein Wasserstoffatom, $R_7$ eine Methylcarboxylatgruppe und $R_8$ tert.-Butyl-carboxylatgruppe bedeuten.